# EUROPÄISCHE PATENTSCHRIFT

(11) **EP 4 277 547 B1**
(45) Veröffentlichungstag und Bekanntmachung des Hinweises auf die Patenterteilung: **24.07.2024**
(21) Anmeldenummer: 22711142.4
(22) Anmeldetag: 12.01.2022
(51) Int. Cl.: A61B 10/00, A61B 5/00, A61B 17/20

(54) **ALLERGIETEST-VORRICHTUNG**
ALLERGY TEST DEVICE
DISPOSITIF DE TEST D'ALLERGIE

(30) Priorität: 15.01.2021 AT 500102021
(43) Veröffentlichungstag der Anmeldung: 22.11.2023
(73) Patentinhaber: ALLTEST GmbH, 4020 Linz (AT)
(72) Erfinder: SCHNETZ, Guntram, 2362 Biedermannsdorf (AT)
(74) Vertreter: Burgstaller, Peter
(86) Internationale Anmeldenummer: PCT/AT2022/060005
(87) Internationale Veröffentlichungsnummer: WO 2022/150861

(56) Entgegenhaltungen:
- WO-A2-2011/053019
- US-A1- 2015 126 898

## Beschreibung

Die Erfindung betrifft eine Allergietest-Vorrichtung zur Aufbringung eines flüssigen Mediums auf oder in die Haut, mit einer Folie mit einem flachen Teil zur Auflage auf der Haut und zumindest einem bei der Anwendung von der Haut beabstandeten Teil mit einem Behälter zur Aufnahme des flüssigen Mediums, wobei jeder Behälter jedes beabstandeten Teils zumindest eine verschließbare Öffnung aufweist.

Die vorliegende Erfindung bezieht sich auf Allergietest-Vorrichtungen mit mindestens einem beabstandeten Teil mit zumindest einem Behälter zur Aufnahme eines flüssigen Mediums, insbesondere aber mit mehreren beabstandeten Teilen mit mehreren Behältern zur Aufnahme verschiedener flüssiger Medien. Dadurch können Allergietests für mehrere Allergene gleichzeitig in besonders effizienter und für den Patienten oder Probanden zeitsparenden Weise durchgeführt werden.

Die vorliegende Erfindung betrifft Allergietest-Vorrichtungen, die zum Testen einer allergischen Reaktion bei bloßer Aufbringung des jeweiligen flüssigen Mediums auf die Oberfläche der Haut ohne Verletzung der Haut verwendet werden. Beispielsweise kann auf diese Weise eine allergische Reaktion auf bestimmte Kosmetika, wie Salben oder Cremen, bestimmt werden. Ebenso umfasst sind Allergietest-Vorrichtung zur Durchführung eines sogenannten Pricktests, bei dem die Haut aufgestochen oder eingeritzt wird, sodass das zu testende flüssige Medium in die Haut eindringen und gegebenenfalls eine allergische Reaktion hervorrufen kann.

Unter den Begriff der flüssigen Medien fallen verschiedenste Flüssigkeiten, welche Inhaltsstoffe enthalten, die hinsichtlich einer allergischen Reaktion des Patienten oder Probanden untersucht werden sollen. Beispielsweise können derartige Inhaltsstoffe definierte Allergenextrakte, wie zum Beispiel Pollenextrakte, Nahrungsmittelextrakte, etc. sein. Für die Anwendung eignen sich besonders dünnflüssige Medien, es ist aber auch die Anwendung zähflüssiger oder gelförmiger Medien denkbar, sofern diese auf die Haut oder in die Haut aufbringbar sind. An geeigneten Stellen der Allergietest-Vorrichtung können auch Bestandteile in fester Form, beispielsweise gefriergetrocknete Allergene in Form eines Lyophilisats, angeordnet sein, welche bei der Anwendung der Allergietest-Vorrichtung mit dem flüssigen Medium vermischt werden. Das Gemisch aus zwei oder mehr Komponenten wird dann auf die Haut oder in die Haut aufgebracht.

Früher wurden beim Pricktest in sehr aufwändiger und zeitintensiver Weise verschiedene Allergene manuell nebeneinander auf die Haut beispielsweise des Unterarms oder Rückens aufgetropft und die Haut angestochen, sodass die jeweilige Substanz in die Oberhaut eindringen kann. Nach einer vorgegebenen Testdauer wurde die allergische Reaktion durch Bestimmung der Hautrötung und Quaddelgröße beurteilt. Parallel wird meist eine Positivkontrolle mit Histamin und eine wirkstofffreie Negativkontrolle durchgeführt. Neben dem großen zeitlichen Aufwand für das medizinische Personal und den Patienten oder Probanden ist es insbesondere bei sehr jungen Patienten oder Probanden sehr schwierig, die Behandlungsdauer ohne Bewegung auszuharren. Darüber hinaus besteht insbesondere bei Bewegungen des Patienten oder Probanden die Gefahr, dass die aufgetropften flüssigen Medien verwischt oder vermischt werden.

Mithilfe von Allergietest-Vorrichtungen, welche bereits die zu untersuchenden flüssigen Medien enthalten, wurde die Durchführung derartiger Allergietests oder Pricktests sowohl für das medizinische Personal als auch den Patienten oder Probanden erheblich erleichtert.

Beispielsweise beschreibt die US 2014/276196 A1 eine Allergietest-Vorrichtung in Form einer Folie mit mehreren Behältern zur Aufnahme verschiedener flüssiger Medien, welche auf die Haut aufgeklebt wird. Nach dem Aufkleben wird mithilfe einer Wippe an der Oberseite der Folie ein Druck über allen Behältern ausgeübt, wodurch die Unterseite der Folie durchstochen, gleichzeitig die oberste Schicht der Haut aufgestochen und das flüssige Medium auf die Einstichstelle aufgebracht wird.

Andere Konstruktionen von Allergietest-Vorrichtungen sind beispielsweise aus der US 2015/126898 A1, der US 2,841,138 A oder der EP 2 163 203 A1 bekannt geworden.

Eine Allergietest-Vorrichtung in Form eines Blisterstreifens ist in der AT 515 791 B1 beschrieben, wobei zwei Folien vorgesehen sind und das jeweilige flüssige Medium in Ausstülpungen der oberen Folie enthalten ist. Durch manuellen Druck auf die Ausstülpungen wird das darin enthaltene Medium durch eine Öffnung auf die Hautoberfläche gepresst und gegebenenfalls gleichzeitig die Haut mit spitzen oder scharfen Elementen geritzt oder punktuell penetriert, sodass das Medium in die Haut eindringen und dort allenfalls eine allergische Reaktion hervorrufen kann.

Bekannte Vorrichtungen zur Durchführung von Allergietests ermöglichen meist keine definierte Aufbringung des jeweiligen Mediums auf die Haut oder in die Haut, da die Öffnung des das Medium aufnehmenden Behälters stark von der Richtung und Höhe der ausgeübten Druckkraft abhängt. Somit resultiert keine gute Reproduzierbarkeit der Allergietests.

Darüber hinaus sind viele Allergietest-Vorrichtungen sehr kompliziert aufgebaut und aufwändig in ihrer Herstellung oder auch in der Methode zur Befüllung mit den flüssigen Medien, wodurch die Kosten steigen.

Die Aufgabe der vorliegenden Erfindung besteht daher darin, eine oben genannte Allergietest-Vorrichtung zu schaffen, welche sowohl möglichst einfach und kostengünstig herstellbar ist, und aus möglichst wenigen Komponenten besteht. Darüber hinaus soll eine einwandfreie Funktion und Reproduzierbarkeit der Allergietests gewährleistet sein und das in den Behältern enthaltene flüssige Medium in sicherer und definierter Weise auf die Oberfläche der darunterliegenden Haut aufgebracht werden können, sodass verlässliche Aussagen über allfällige allergische Reaktionen getroffen werden können. Nachteile bekannter Allergietest-Vorrichtungen sollen vermieden oder zumindest reduziert werden.

Gelöst wird die erfindungsgemäße Aufgabe dadurch, dass jeder beabstandete Teil in Richtung des flachen Teils der Folie verformbar ausgebildet ist und dass in der zumindest einen Öffnung jedes Behälters ein Ventil verschiebbar angeordnet ist, wobei die Öffnung des Behälters in einer Schließposition des Ventils abgedichtet ist und bei Verformung des beabstandeten Teils das Ventil in einer von der Schließposition abweichenden Position zumindest teilweise geöffnet ist. Die erfindungsgemäße Allergietest-Vorrichtung besteht aus wenigen Komponenten, welche relativ einfach, beispielsweise im Spritzgussverfahren aus geeigneten Kunststoffen herstellbar sind. Unter der Voraussetzung der Herstellung entsprechend hoher Stückzahlen können auch relativ niedrige Herstellungskosten erzielt werden. Durch die Anordnung eines Ventils in der Öffnung jedes Behälters mit dem flüssigen Medium kann bei Druck auf den beabstandeten Teil der Folie in Richtung Hautoberfläche eine definierte Öffnung zumindest eines Teils der Öffnung des Behälters erfolgen und somit eine sichere Aufbringung des flüssigen Mediums und allenfalls damit vermischter Stoffe auf die Hautoberfläche. Durch entsprechende Konstruktion des Ventils und der Öffnung des Behälters kann eine Abdichtung des Behälters in der Schließposition des Ventils erreicht werden und dadurch das im Behälter befindliche flüssige Medium auch über längere Zeiträume sicher aufbewahrt werden. Je nach verwendetem Medium kann der Behälter aber auch erst kurz vor der Durchführung des Allergietests damit befüllt werden. Idealerweise werden die Allergietest-Vorrichtungen aber bereits mit dem flüssigen Medium und allenfalls davon getrennten festen Stoffen, wie zum Beispiel gefriergetrockneten Allergenen (Lyophilisaten), welche bei der Anwendung mit dem flüssigen Medium vermischt werden, vorbefüllt und entsprechend verpackt vertrieben, sodass das medizinische Personal diese nur mehr öffnen und beim Patienten oder Probanden anwenden muss.

Vorzugsweise ist der Behälter im beabstandeten Teil der Folie integriert. Dadurch können die Herstellungskosten noch weiter minimiert werden, da sowohl die Folie als auch der Behälter aus einem Material und vorzugsweise auch aus einem Stück hergestellt werden kann.

Im Falle eines im beabstandeten Teil der Folie integrierten Behälters kann in jedem beabstandeten Teil eine vorzugsweise mit einem Deckel verschließbare Befüllöffnung zur Befüllung des Behälters mit dem flüssigen Medium angeordnet sein. Auf diese Weise wird zwar der Herstellungsprozess komplexer und somit die Herstellungskosten höher, allerdings die Befüllbarkeit der Behälter der Allergietest-Vorrichtung mit dem jeweiligen flüssigen Medium wesentlich erleichtert. Das Medium könnte anstatt über eine Befüllöffnung beispielsweise auch mit Hilfe einer Spritze durch eine Einstechmembran in den Behälter eingebracht werden.

Alternativ zu der oben beschriebenen Integration des Behälters im beabstandeten Teil der Folie kann der Behälter auch separat im Wesentlichen in Form eines Bechers hergestellt und nachträglich mit dem beabstandeten Teil der Folie entsprechend verbunden werden. Diese Variante erleichtert die Herstellung und Formung der Folie, erfordert aber einen zusätzlichen Herstellungsschritt der Verbindung des Behälters mit dem beabstandeten Teil der Folie. In diesen zusätzlichen Herstellungsschritt kann jedoch der Befüllvorgang des Behälters mit dem jeweiligen flüssigen Medium in idealer Weise integriert werden.

Vorzugsweise ist zumindest jeder beabstandete Teil der Folie, vorzugsweise die gesamte Folie elastisch verformbar ausgebildet. Dadurch wird gewährleistet, dass der beabstandete Teil nach Ausübung des Drucks wieder in seine ursprüngliche Lage zurückbewegt wird und dadurch die Komponenten der Allergietest-Vorrichtungen wieder von der Hautoberfläche wegbewegt werden, nachdem das flüssige Medium oder eine Mischung des flüssigen Mediums mit festen Stoffen aus dem Behälter auf die Haut aufgebracht oder in die Haut eingebracht wurde. Somit kann die allfällige allergische Reaktion auf das jeweilige Medium während der Durchführung des Allergietests ungehindert ablaufen und ein optimales Ergebnis erzielt werden.

Die Folie ist vorzugsweise aus Kunststoff, insbesondere aus thermoplastischem Kunststoff oder Silikon, gebildet. Diese Materialien sind leicht verarbeitbar und können auch die Voraussetzungen für Biokompatibilität erfüllen. Außerdem kann die elastische Verformbarkeit, also die Rückkehr in die ursprüngliche Form oder zumindest im Wesentlichen ursprüngliche Form nach einer Verformung mit derartigen Materialien erzielt werden.

Jeder beabstandete Teil kann durch eine Ausstülpung der Folie gebildet sein, sodass bei der Anwendung ein vorzugsweise abgeschlossener Hohlraum zur Haut gebildet ist. Dadurch kann ein Vermischen der flüssigen Medien verschiedener Ausstülpungen während des Allergietests und eine Beeinflussung der Untersuchung durch äußere Einflüsse verhindert werden. Unter der Voraussetzung einer geeigneten Konstruktion kann sogar dadurch eine Bewegung des Patienten oder Probanden während des Allergietests in gewissen Grenzen ermöglicht werden, da das flüssige Medium nicht unkontrolliert verrinnen kann.

Um die Entleerung des Behälters in den Hohlraum unter der Ausstülpung der Folie nicht zu behindern, kann in der Ausstülpung auch ein Entlüftungsloch angeordnet sein. Über das Entlüftungsloch, welches allenfalls ein Ventil enthalten kann, sodass keine Luft von Außen in den Hohlraum gelangen kann, entweicht beim Zusammendrücken der Ausstülpung Luft, wodurch das flüssige Medium ungehindert aus dem Behälter auf die Hautoberfläche rinnen kann.

Zur Durchführung eines sogenannten Pricktests ist an der, bei der Anwendung der Haut zugewandten Seite des Behälters und bzw. oder des Ventils zumindest ein spitzes oder scharfes Element zur lokalen und oberflächlichen Verletzung der Haut vorgesehen. Das zumindest eine spitze oder scharfe Element ist an einer geegneten Stelle oder geeigneten Stellen der Unterseite des Behälters und bzw. oder des Ventils angeordnet, wodurch während der Verformung des beabstandeten Teils der Folie durch entsprechende Druckausübung gleichzeitig mit dem Öffnen des Ventils auch das Perforieren oder Ritzen der Haut durchgeführt wird.

Vorteilhafterweise ist das zumindest eine spitze oder scharfe Element einstückig mit dem Behälter und bzw. oder dem Ventil hergestellt. Beispielsweise kann das spitze oder scharfe Element im Spritzgussverfahren beim Produzieren des Behälters oder Ventils integriert sein, wodurch die Herstellungskosten sehr gering gehalten werden können. Für spezielle Anwendungen kann es auch notwendig sein, spitze oder scharfe Elemente aus Edelstahl zu verwenden, welche auch nachträglich am Behälter oder Ventil angeordnet werden können, wodurch aber die Herstellungskosten erhöht werden.

Gemäß einem weiteren Merkmal der Erfindung ist jedes Ventil durch einen im Wesentlichen zylindrischen Körper mit zumindest einem Kanal gebildet, welcher zylindrische Körper in der Schließposition des Ventils die Öffnung des Behälters abdichtet und in einer von der Schließposition abweichenden Position den zumindest einen Kanal zum Behälter freigibt. Ein derartiger zylindrischer Körper ist relativ leicht herstellbar.

Dabei kann der zumindest eine Kanal durch zumindest einen im Inneren des zylindrischen Körpers verlaufenden Kanal mit einer Verbindung zu mindestens einer Öffnung an der Mantelfläche gebildet sein. In der Schließstellung ist die Öffnung des im Inneren verlaufenden Kanals an der Mantelfläche verschlossen und es kann kein flüssiges Medium vom Behälter nach außen dringen. Bei Verschiebung des Ventils aus der Schließposition wird die zumindest eine Öffnung an der Mantelfläche des zylindrischen Körpers freigegeben, wodurch das flüssige Medium über diese Öffnung durch den im Inneren verlaufenden Kanal zur Hautoberfläche gelangen kann. Darüber hinaus kann im Inneren des zylindrischen Körpers ein fester Stoff angeordnet oder das Innere des zylindrischen Körpers mit einer Beschichtung eines festen Stoffes, beispielsweise einem gefriergetrocknetem Allergen (Lyophilisat) versehen sein, welcher feste Stoff während der Anwendung der Allergietest-Vorrichtung mit dem flüssigen Medium vermischt und auf die Haut aufgebracht oder in die Haut eingebracht wird.

Alternativ oder zusätzlich kann der zumindest eine Kanal auch durch zumindest einen an der Oberfläche des zylindrischen Körpers verlaufenden Kanal gebildet sein. In der Schließposition des Ventils wird die Öffnung des Behälters durch entsprechende Konstruktionen des Ventils und der Öffnung abgedichtet und ein Austreten des flüssigen Mediums aus dem Behälter verhindert. Bei Verschiebung des Ventils gegenüber der Schließposition wird dann der zumindest eine an der Oberfläche befindliche Kanal am zylindrischen Körper freigegeben und das flüssige Medium fließt vom Behälter über den zumindest einen außen an der Oberfläche des Ventils verlaufenden Kanal in Richtung Haut.

Am zylindrischen Körper des Ventils kann eine Dichtlippe zur Abdichtung der Öffnung des Behälters in der Schließposition integriert sein. Eine derartige Dichtlippe kann beim Herstellungsprozess des Ventils, beispielsweise dem Spritzgussverfahren, einfach integriert werden. Die Dichtlippe verhindert zuverlässig ein ungewolltes Auslaufen des flüssigen Mediums aus dem Behälter und ein ungewolltes Vermischen des flüssigen Mediums mit einem allenfalls vorhandenen festen Stoff, wenn sich das Ventil in der Schließposition befindet.

An der bei der Anwendung der Haut zugewandten Seite des flachen Teils der Folie ist vorzugsweise eine Klebefolie mit einer Abziehfolie angeordnet. Somit kann die entsprechend vorgefertigte Allergietest-Vorrichtung durch einfaches Abziehen der Abziehfolie und Aufkleben an einer geeigneten Hautstelle des Patienten oder Probanden rasch angewendet werden. Anstelle einer vorgefertigten Klebefolie kann die Allergietest-Vorrichtung beispielsweise auch durch Aufbringen, insbesondere Aufsprühen eines geeigneten Klebemittels an die Unterseite der flachen Teile der Folie für die Anwendung am Patienten oder Probanden vorbereitet werden. Darüber hinaus kann die Allergietest-Vorrichtung auch einfach mit Klebestreifen an der Hautoberfläche befestigt und fixiert werden.

Gemäß einem weiteren Merkmal der Erfindung sind mehrere beabstandete Teile einer Folie in zumindest zwei Reihen angeordnet. Somit kann eine allergische Reaktion auf verschiedene Substanzen, welche in den flüssigen Medien der verschiedenen Behälter in den beabstandeten Teilen der Folie enthalten sind, gleichzeitig erfolgen.

Um eine eindeutige Zuordnung der jeweiligen flüssigen Medien in den Behältern der einzelnen beabstandeten Teile zu ermöglichen, ist die Anzahl der beabstandeten Teile einer Folie in jeder Reihe unterschiedlich. Somit kann einer Vertauschung der Reihen bei der Beurteilung der allergischen Reaktionen verhindert und eine sichere und eindeutige Zuordnung der jeweiligen flüssigen Medien zu den entsprechenden Hautarealen erzielt werden.

An der Außenseite jedes beabstandeten Teils ist vorzugsweise eine Identifikation angeordnet. Beispielsweise sind an der Außenseite der beabstandeten Teile oder Ausstülpungen der Folie Ziffern oder Buchstaben angeordnet, wobei die flüssigen Medien diesen Ziffern oder Buchstaben in einer begleitenden Legende zugeordnet werden. Dadurch weiß das medizinische Personal, welches flüssige Medium in welchem Behälter welchen beabstandeten Teils der Folie enthalten ist und kann nach Durchführung des Allergietest eine allergische Reaktion rasch und einfach dem jeweiligen flüssigen Medium bzw. der darin gelösten Substanz zuordnen.

Die Folie kann zumindest teilweise aus transparentem Material, beispielsweise aus transparentem thermoplastischem Kunststoff oder transparentem Silikon, gebildet sein. Dadurch kann einerseits die Füllung der Behälter und andererseits die korrekte Durchführung des Allergietests in Augenschein genommen und überprüft werden. Wenn nicht alle Teile aus transparentem Material hergestellt werden, können diese beispielsweise in einem Zweikomponenten-Spritzgussverfahren nach der Herstellung der nichttransparenten Teile hergestellt werden.

Wenn eine Transportsicherung vorgesehen ist, mit im Wesentlichen zylinderförmigen Elementen, welche zur Anordnung an der Unterseite der Folie zur Umfassung der Behälter und Ventile in den beabstandeten Teilen ausgebildet ist, kann eine ungewollte Verschiebung der Ventile bei Verformung der beabstandeten Teile der Folie und somit ein ungewolltes Öffnen der Behälter und Austreten der flüssigen Medien aus den Behältern und eine Vermischung des flüssigen Mediums mit allenfalls vorliegenden festen Stoffen verhindert werden. Die Transportsicherung kann auch sehr einfach und billig, vorzugsweise im Spritzgussverfahren, hergestellt werden. Die Allergietest-Vorrichtung wird vorzugsweise zusammen mit einer solchen Transportsicherung und vorzugsweise in einer Verpackung ausgeliefert und die Transportsicherung erst kurz vor der Anwendung der Allergietest-Vorrichtung und kurz vor dem Aufkleben der Vorrichtung auf der Hautoberfläche des Patienten oder Probanden entfernt.

Wenn an der Transportsicherung ein Lineal, vorzugsweise mit halbkreisförmigen Aussparungen mit Maßlinien, zur Vermessung allergischer Reaktionen auf der Haut angeordnet ist, kann die Auswertung des Allergietests vereinfacht bzw. unterstützt werden. Das medizinische Personal kann das Lineal nach dem Entfernen der Allergietest-Vorrichtung von der Hautoberfläche des Patienten oder Probanden dazu verwenden, um die Größe der bei der allergischen Reaktion entstehenden Quaddeln zu bestimmen. Zu diesem Zweck sind beispielsweise mehrere halbkreisförmige Aussparungen auf dem Lineal mit verschiedenen Durchmessern vorgesehen, welche in Übereinstimmung mit der allergischen Reaktion gebracht werden, um die Größe der kreisförmigen Rötung ablesen zu können. Auch verschiedene Rotfarbtöne zur Quantifizierung der Hautrötung könnten am Lineal der Transportsicherung aufgedruckt sein, um die Diagnose des medizinischen Personals zu erleichtern.

Wenn eine vorzugsweise sterile Verpackung vorgesehen ist, kann die Allergietest-Vorrichtung bis zur Befüllung mit den flüssigen Medien und allenfalls festen Stoffen oder bei bereits befüllten Behältern auch bis zur Anwendung am Patienten bzw. Probanden sauber bzw. steril gehalten werden. Um die Sicht auf die Allergietest-Vorrichtung in der Verpackung zu ermöglichen, ist die Verpackung zumindest teilweise transparent ausgebildet. Die allfällige Sterilisation der Allergietest-Vorrichtung kann mit geeigneten Sterilisationsverfahren, wie zum Beispiel mit Gas, Gammastrahlung, UV-Licht, etc. erfolgen.

Die Folie, der Behälter, das Ventil und bzw. oder das zumindest eine spitze oder scharfe Elemente sind vorzugsweise zumindest teilweise aus biokompatiblem Material, beispielsweise aus Polyetheretherketon (PEEK), gebildet oder zumindest teilweise mit einer biokompatiblen Beschichtung, beispielsweise einer Parylene-Beschichtung, beschichtet.

Die vorliegende Erfindung wird anhand der beigefügten Zeichnungen näher erläutert. Darin zeigen
- Fig. 1A: eine schematische Prinzipdarstellung der erfindungsgemäßen Allergietest-Vorrichtung in geschnittener Seitenansicht in Schließposition des Ventils, in der die Öffnung des Behälters abgedichtet ist;
- Fig. 1B: die Allergietest-Vorrichtung gemäß Fig. 1A in einer von der Schließposition des Ventils abweichenden Position, in der die Öffnung des Behälters zur Freigabe des flüssigen Mediums zumindest teilweise geöffnet ist;
- Fig. 2: ein Schnittbild durch eine Ausführungsform der erfindungsgemäßen Allergietest-Vorrichtung mit einem in der Folie integriertem Behälter;
- Fig. 3A: ein Schnittbild durch eine weitere Ausführungsform der erfindungsgemäßen Allergietest-Vorrichtung in Schließposition des Ventils;
- Fig. 3B: die Allergietest-Vorrichtung gemäß Fig. 3A in einer von der Schließposition des Ventils abweichenden Position zur Freigabe des flüssigen Mediums;
- Fig. 4A: ein Schnittbild durch eine weitere Ausführungsform der erfindungsgemäßen Allergietest-Vorrichtung in Schließposition des Ventils;
- Fig. 4B: die Allergietest-Vorrichtung gemäß Fig. 4A in einer von der Schließposition des Ventils abweichenden Position zur Freigabe des flüssigen Mediums;
- Fig. 5: eine perspektivische Ansicht auf eine Transportsicherung mit integriertem Lineal zum Vermessen der allergischen Reaktion;
- Fig. 6: ein Schnittbild durch eine Ausführungsvariante der Allergietest-Vorrichtung mit daran angeordneter Transportsicherung;
- Fig. 7: das Detail VII aus Figur 6 in vergrößerter Darstellung;
- Fig. 8: eine perspektivische Draufsicht auf eine Ausführungsform einer erfindungsgemäßen Allergietest-Vorrichtung von oben; und
- Fig. 9: eine perspektivische Ansicht auf eine in einer Verpackung angeordnete Allergietest-Vorrichtung.

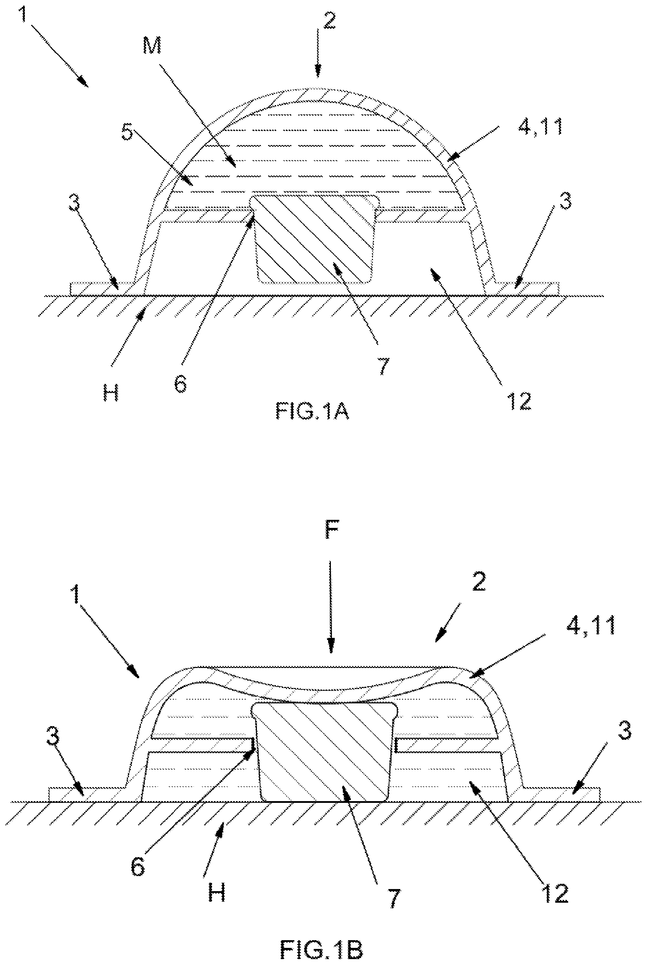

Fig. 1A zeigt eine schematische Prinzipdarstellung der erfindungsgemäßen Allergietest-Vorrichtung 1 zur Aufbringung eines flüssigen Mediums M auf oder in die Haut H eines Patienten oder Probanden. Die Allergietest-Vorrichtung 1 weist eine Folie 2 mit einem flachen Teil 3 zur Auflage auf der Haut H und einem bei der Anwendung von der Haut H beabstandeten Teil 4 auf. Die Folie 2 kann zumindest teilweise aus transparentem Material, beispielsweise aus transparentem thermoplastischem Kunststoff oder transparentem Silikon, gebildet sein. Jeder beabstandete Teil 4 der Folie 2 weist einen Behälter 5 zur Aufnahme des flüssigen Mediums M auf, dessen allergische Reaktion getestet werden soll. Das Medium M kann auch zähflüssig, pastös oder gelförmig sein. Jeder Behälter 5 im beabstandeten Teil 4 weist zumindest eine verschließbare Öffnung 6 auf, über welche das flüssige Medium M auf die Haut H aufgebracht oder in die Haut H eingebracht werden kann. Der flache Teil 3 der Folie 2 kann mit einem Klebstoff oder einer Klebefolie (nicht dargestellt) auf der Oberfläche der Haut H fixiert werden. Erfindungsgemäß ist jeder beabstandete Teil 4 in Richtung des flachen Teils 3 der Folie 2 verformbar ausgebildet in der zumindest einen Öffnung 6 jedes Behälters 5 ein Ventil 7 verschiebbar angeordnet. In Figur 1A befindet sich das Ventil 7 in seiner Schließposition, in der die Öffnung 6 des Behälters 5 abgedichtet ist und ein Austritt des flüssigen Mediums M aus dem Behälter 5 verhindert wird.

Wird nun gemäß Fig. 1B durch Aufbringung einer Kraft F auf den beabstandeten Teil 4 der Folie 2 eine Verformung des beabstandeten Teils 4 und des Behälters 5 bewirkt, wird das Ventil 7 in eine von der Schließposition gemäß Fig. 1A abweichende Position gebracht, sobald es an der Oberfläche der Haut H ansteht und dadurch ins Innere des Behälters 5 verschoben wird. Durch die Verschiebung des Ventils 7 wird die Öffnung 6 des Behälters 5 zumindest teilweise geöffnet, wodurch das flüssige Medium aus dem Behälter 5 auf die Oberfläche der Haut H austreten kann. Im dargestellten schematischen Beispiel ist das Ventil 7 durch einen Kegelstumpfförmigen Pfropfen gebildet, wodurch bei Verschiebung aus der Schließposition ein ringförmiger Spalt zwischen Ventil 7 und Öffnung 6 freigegeben wird, durch welchen das flüssige Medium M aus dem Behälter 5 austreten kann. Wenn die Einwirkung der Kraft F wieder aufgegeben wird, kehrt der beabstandete Teil 4 der Folie 2 vorzugsweise wieder in die ursprüngliche Form gemäß Fig. 1A zurück. Zu diesem Zweck ist zumindest der beabstandete Teil 4 der Folie 2, vorzugsweise die gesamte Folie 2 aus elastisch verformbarem Material gebildet. Dadurch wird verhindert, dass Komponenten der Allergietest-Vorrichtung 1, wie zum Beispiel das Ventil 7, auf der Oberfläche der Haut H aufliegen und die allergische Reaktion auf das flüssige Medium M verhindern oder beeinflussen.

Eine solche Allergietest-Vorrichtung 1 kann relativ einfach hergestellt werden, vorzugsweise aus geeigneten Kunststoffen im Spritzgussverfahren. Durch die Anordnung des Ventils 7 in der Öffnung 6 des Behälters 5 mit dem flüssigen Medium M kann bei Druck auf den beabstandeten Teil 4 der Folie 2 in Richtung Oberfläche der Haut H eine definierte Öffnung zumindest eines Teils der Öffnung 6 des Behälters 5 erfolgen und somit eine sichere Aufbringung des flüssigen Mediums M auf die Hautoberfläche erzielt werden. Bei der dargestellten Ausführungsvariante gemäß Fig. 1A und Fig. 1B ist der Behälter 5 in der Folie 2 bzw. im beabstandeten Teil 4 der Folie 2 integriert und mit dieser einstückig hergestellt. Hier ist der beabstandete Teil 4 durch eine Ausstülpung 11 der Folie 2 gebildet, sodass bei der Anwendung der Allergietest-Vorrichtung 1 ein vorzugsweise abgeschlossener Hohlraum 12 zur Haut H gebildet wird. Der beabstandete Teil 4 der Folie 2 muss aber nicht zwingend abgeschlossen sein.

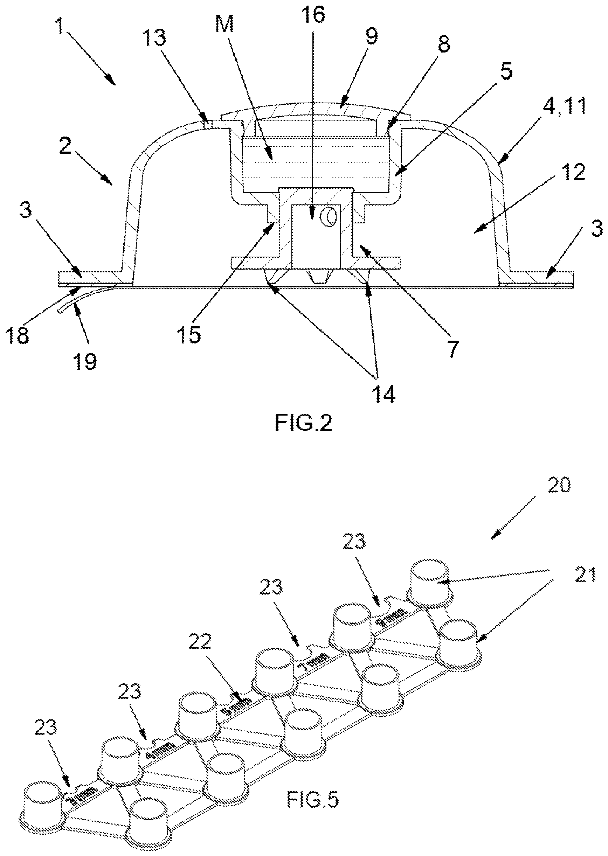

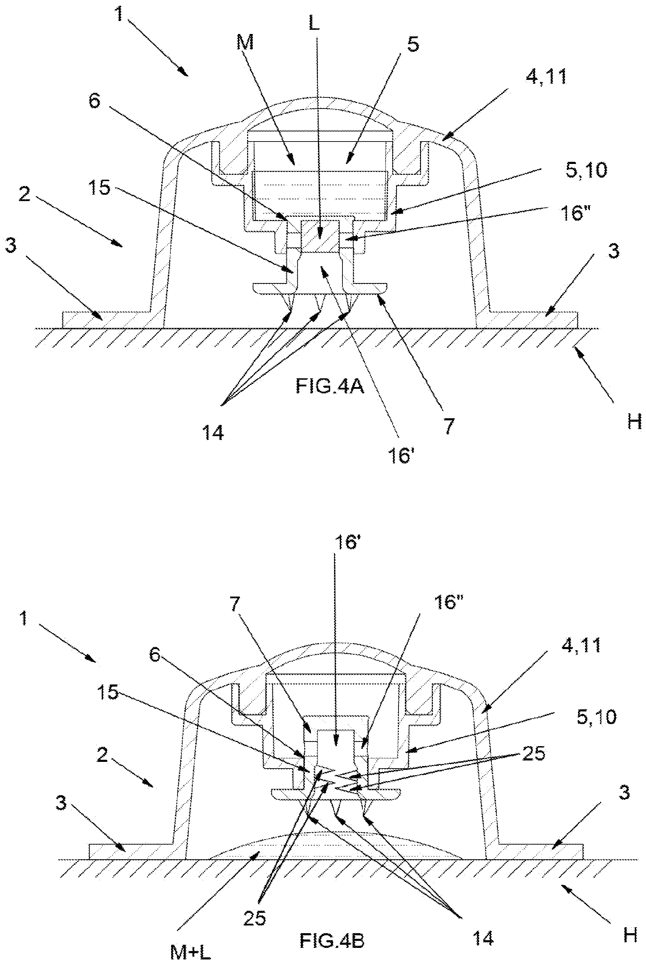

Fig. 2 zeigt ein Schnittbild durch eine andere Ausführungsform der erfindungsgemäßen Allergietest-Vorrichtung 1 mit einem in der Folie 2 integriertem Behälter 5. Der beabstandete Teil 4 der Folie ist auch hier in Form einer Ausstülpung 11 gebildet, wodurch sich ein abgeschlossener Hohlraum 12 zur Hautoberfläche bildet. Der Behälter 5 ist in Form eines Bechers aus dem beabstandeten Teil 4 der Folie 2 gebildet und weist eine mit einem Deckel 9 verschließbare Befüllöffnung 8 zur Befüllung des Behälters 5 mit dem flüssigen Medium M auf. Im beabstandeten Teil 4 der Folie 2 bzw. in der Ausstülpung 11 kann auch ein Entlüftungsloch 13, allenfalls mit einem Entlüftungsventil (nicht gezeigt) vorgesehen sein. Das Ventil 7 ist durch einen im wesentlichen zylindrischen Körper 15 mit einem darin verlaufenden Kanal 16 gebildet (siehe Fig. 4A und 4B). An der, bei der Anwendung der Haut H zugewandten Seite des Ventils 7 sind spitze oder scharfe Elemente 14 zur lokalen und oberflächlichen Verletzung der Haut H vorgesehen, welche vorzugsweise einstückig mit dem Ventil 7 hergestellt sind. An der bei der Anwendung der Haut H zugewandten Seite des flachen Teils 3 der Folie 2 kann eine Klebefolie 18 mit einer Abziehfolie 19 angeordnet sein, wie schematisch angedeutet.

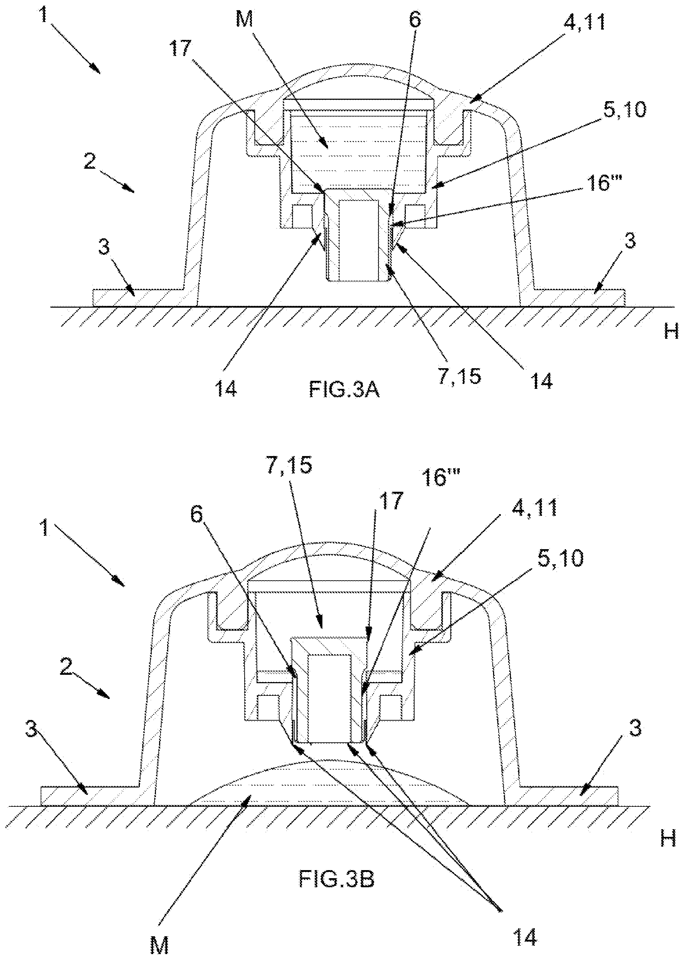

In Fig. 3A ist ein Schnittbild durch eine weitere Ausführungsform der erfindungsgemäßen Allergietest-Vorrichtung 1 in Schließposition des Ventils 7 dargestellt. Hier ist der Behälter 5 durch einen eigenständigen Körper im Wesentlichen in Form eines Bechers 10 gebildet und mit dem beabstandeten Teil 4 der Folie bzw. der Ausstülpung 11 entsprechend dicht verbunden, beispielsweise verklebt. In der Öffnung 6 des Behälters 5 ist ein Ventil 7 angeordnet, welches durch einen im wesentlichen zylindrischen Körper 15 mit Kanälen 16‴ an der Oberfläche des zylindrischen Körpers 15 gebildet ist. Diese an der Oberfläche des das Ventil 7 bildenden zylindrischen Körpers 15 können durch eine oder mehrere axiale Rillen gebildet sein. In der Schließposition des Ventils 7 ist die Öffnung 6 des Behälters 5 durch eine Erweiterung des Durchmessers des zylindrischen Körpers 15 abgedichtet. Zusätzlich oder alternativ dazu kann am oberen, der Haut H abgewendeten Ende des zylindrischen Körpers 15 auch eine Dichtlippe 17 angeordnet sein, welche insbesondere in einem Spritzgussverfahren einfach herstellbar ist.

Wird gemäß Fig. 3B das Ventil 7 durch Verformung des beabstandeten Teils 4 der Folie 2 aus der Schließposition gemäß Fig. 3A verschoben, werden die Kanäle 16‴ an der Außenseite des zylindrischen Körpers 15 des Ventils 7 freigegeben und das flüssige Medium kann vom Behälter 5 auf die Oberfläche der Haut H gelangen. An der, der Haut H zugewandten Seite des Behälters 5 können spitze oder scharfe Elemente 14 angeordnet sein, welche zum Perforieren oder Ritzen der Haut H dienen. Die spitzen oder scharfen Elemente 14 sind vorzugsweise im Behälter 5 integriert und mit diesem einstückig hergestellt.

Fig. 4A zeigt ein Schnittbild durch eine weitere Ausführungsform der erfindungsgemäßen Allergietest-Vorrichtung 1 in Schließposition des Ventils 7. Gegenüber der Ausführungsform gemäß Fig. 3A und 3B ist das Ventil 7 durch einen im Wesentlichen zylindrischen Körper 15 mit einem Kanal 16' im Inneren des zylindrischen Körpers 15 mit einer Verbindung zu mindestens einer Öffnung 16" an der Mantelfläche des zylindrischen Körpers 15 gebildet. An der, der Haut H zugewandten Seite des Ventils 7 sind spitze oder scharfe Elemente 14 angeordnet und vorzugsweise einstückig mit dem Ventil 7 hergestellt. Zusätzlich ist bei diesem Ausführungsbeispiel im Kanal 16' im Inneren des zylindrischen Körpers 15 ein Lyophilisat L (beispielsweise ein gefriergetrocknetes Allergen) angeordnet, welches sich bei Verschiebung des Ventils 7 aus der Schließposition (siehe Fig. 4B) mit dem flüssigen Medium M vermischt und dadurch aktiviert wird. Das Lyophilisat L kann vorgeformt gepresst, chemisch/physikalisch gebunden oder eingekapselt vorliegen. Anstelle eines vorgeformten Pfropfens des Lyophilisats L ist auch eine Beschichtung der Innenwand des Kanals 16' oder anderer geeigneter Stellen der Allergietest-Vorrichtung 1 mit dem Lyophilisat L denkbar (nicht dargestellt).

Wird nun gemäß Fig. 4B das Ventil 7 durch Verformung des beabstandeten Teils 4 der Folie 2 aus der Schließposition gemäß Fig. 4A verschoben, werden die Öffnungen 16" an der Mantelfläche des zylindrischen Körpers 15 des Ventils 7 freigegeben und das flüssige Medium kann vom Behälter 5 über die Öffnungen 16" in den im Inneren des zylindrischen Körpers 15 verlaufenden Kanal 16' dringen, wo das flüssige Medium M mit dem Lyophilisat L vermischt wird. Zur Unterstützung der Vermischung des flüssigen Mediums M mit dem Lyophilisat L können Ausformungen 25 an der Innenwand des zylindrischen Körpers 15 angeordnet sein. Danach gelangt die Mischung des flüssigen Mediums M mit dem Lyophilisat L auf die Haut H bzw. in die Haut H. An der, der Haut H zugewandten Seite des Ventils 7 sind die spitzen oder scharfen Elemente 14 angeordnet, welche zum Perforieren oder Ritzen der Haut H dienen.

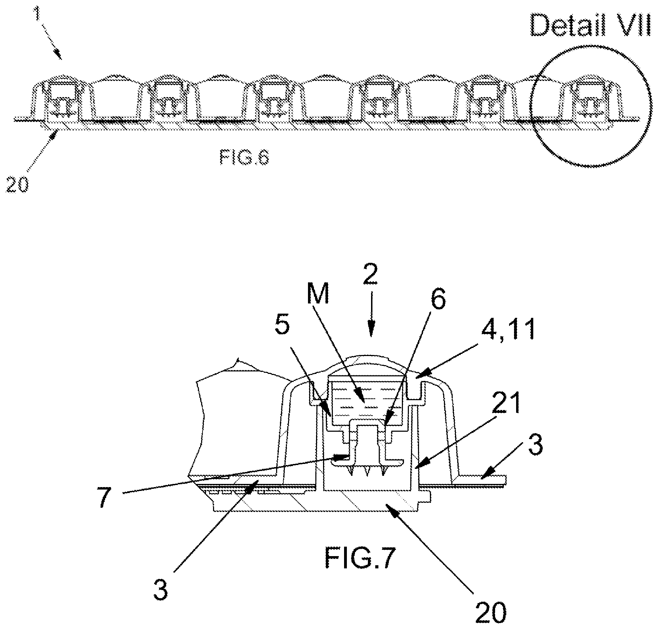

In Fig. 5 ist eine perspektivische Ansicht auf eine Transportsicherung 20 für die Allergietest-Vorrichtung 1 dargestellt. Die Transportsicherung 20 besitzt entsprechend der Anzahl und Anordnung der beabstandeten Teile 4 bzw. Ausstülpungen 11 der Allergietest-Vorrichtung 1 platzierte im Wesentlichen zylinderförmigen Elemente 21, welche zur Anordnung an der Unterseite der Folie 2 zur Umfassung der Behälter 5 und Ventile 7 in den beabstandeten Teilen 4 der Allergietest-Vorrichtung 1 ausgebildet sind (siehe Fig. 6 und 7). An der Transportsicherung 20 kann ein Lineal 22, vorzugsweise mit halbkreisförmigen Aussparungen 23 unterschiedlicher Durchmesser, zur Vermessung allergischer Reaktionen auf der Haut H angeordnet sein. Die jeweiligen Durchmesser der halbkreisförmigen Aussparungen 23 können aufgedruckt werden, um so die Bestimmung der Größe der Quaddeln der allergischen Reaktion zu erleichtern.

Fig. 6 zeigt ein Schnittbild durch eine Ausführungsvariante der Allergietest-Vorrichtung 1 mit daran angeordneter Transportsicherung 20 gemäß Fig. 5.

Fig. 7 zeigt das Detail VII aus Figur 6 in vergrößerter Darstellung. Die im Wesentlichen zylinderförmigen Elemente 21 ragen in die Ausstülpungen 11 der Folie 2 hinein und umgeben die Unterseite der darin befindlichen Behälter 5 mit dem in der Öffnung 6 verschiebbar angeordneten Ventilen 7. Dadurch, dass der beabstandete Teil 4 der Folie auf dem freien Ende des zylinderförmigen Elements 21 aufliegt, wird eine Verformung des beabstandeten Teils 4 und dadurch eine ungewollte Verschiebung des Ventils 7 aus der Schließposition verhindert. Somit kann ein unbeabsichtigtes Ausfließen des flüssigen Mediums M aus dem Behälter wirkungsvoll verhindert werden. Vor Durchführung des Allergietests wird die Transportsicherung 20 von der Allergietest-Vorrichtung 1 gelöst, wodurch die Verformung der beabstandeten Teile 4 der Folie 2 zur Freigabe der flüssigen Medien M auf die Haut H ermöglicht wird. Die Transportsicherung 20 kann einfach auf die Allergietest-Vorrichtung 1 aufgesteckt oder auch mit Klebeverbindungen am flachen Teil 3 der Folie 2 befestigt sein.

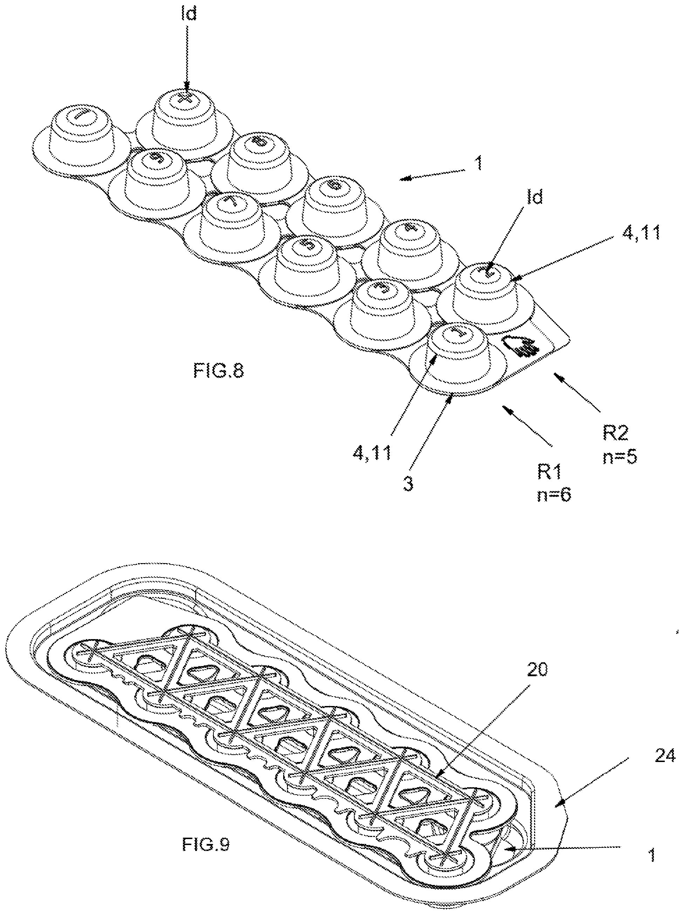

Fig. 8 zeigt eine perspektivische Draufsicht auf eine Ausführungsform einer erfindungsgemäßen Allergietest-Vorrichtung 1 von oben. Dabei sind jeweils mehrere beabstandete Teile 4 der Folie 2 bzw. Ausstülpungen 11 in zwei Reihen R₁ und R₂ angeordnet. Die Anzahl n₁ und n₂ der Ausstülpungen 11 in jeder Reihe R₁ und R₂ ist bewusst unterschiedlich ausgebildet, um eine leichtere und eindeutige Zuordnung der jeweiligen flüssigen Medien M zu ermöglichen und die Diagnose des Allergietests zu erleichtern. Im dargestellten Ausführungsbeispiel sind in der ersten Reihe R₁ sechs (n₁=6) beabstandete Teile 4 mit sechs Behältern 5 für sechs verschiedene flüssige Medien M, also sechs verschiedene Allergietests angeordnet, während in der zweiten Reihe R₂ fünf (n₂=5) beabstandete Teile 4 mit fünf Behältern 5 für fünf flüssige Medien M, also fünf verschiedene Allergietests angeordnet sind. Insgesamt kann also mit dieser Allergietest-Vorrichtung 1 die allergische Reaktion auf elf verschiedene Substanzen gleichzeitig getestet werden bzw. zumindest auf neun verschiedene Substanzen, wenn zwei Behälter 5 für eine Positivkontrolle mit Histamin und eine wirkstofffreie Negativkontrolle reserviert werden.

An der Oberseite der Ausstülpungen 11 kann eine Identifikation Id, beispielsweise eine Ziffer oder ein Buchstabe, zur eindeutigen Zuordnung angeordnet oder ein Beschriftungsfeld platziert sein.

Fig. 9 zeigt eine perspektivische Ansicht auf eine in einer Verpackung 24 angeordnete Allergietest-Vorrichtung 1. Im dargestellten Beispiel ist die Allergietest-Vorrichtung 1 mit daran angeordneter Transportsicherung 20 von unten sichtbar. Die Verpackung 24 ist vorzugsweise zumindest teilweise transparent ausgebildet, um eine Sicht auf die Allergietest-Vorrichtung 1 zu ermöglichen. Die Allergietest-Vorrichtung 1 kann vor der Anordnung in der Verpackung 24 oder auch danach mit gängigen Sterilisationsmethoden sterilisiert werden.

Die einzelnen Komponenten der Allergietest-Vorrichtung 1, wie die Folie 2, die Behälter 5, die Ventile 7 und bzw. oder die spitzen oder scharfen Elemente 14 sind vorzugsweise aus biokompatiblem Material, beispielsweise aus Polyetheretherketon (PEEK), gebildet oder mit einer biokompatiblen Beschichtung, beispielsweise einer Parylene-Beschichtung, beschichtet.

## Patentansprüche

1. Allergietest-Vorrichtung (1) zur Aufbringung eines flüssigen Mediums (M) auf oder in die Haut (H), mit einer Folie (2) mit einem flachen Teil (3) zur Auflage auf der Haut (H) und zumindest einem bei der Anwendung von der Haut (H) beabstandeten Teil (4) mit einem Behälter (5) zur Aufnahme des flüssigen Mediums (M), wobei jeder Behälter (5) jedes beabstandeten Teils (4) zumindest eine verschließbare Öffnung (6) aufweist, **dadurch gekennzeichnet, dass** jeder beabstandete Teil (4) in Richtung des flachen Teils (3) der Folie (2) verformbar ausgebildet ist und dass in der zumindest einen Öffnung (6) jedes Behälters (5) ein Ventil (7) verschiebbar angeordnet ist, wobei die Öffnung (6) des Behälters (5) in einer Schließposition des Ventils (7) abgedichtet ist und bei Verformung des beabstandeten Teils (4) das Ventil (7) in einer von der Schließposition abweichenden Position zumindest teilweise geöffnet ist.

2. Allergietest-Vorrichtung (1) nach Anspruch 1, **dadurch gekennzeichnet, dass** der Behälter (5) im beabstandeten Teil (4) der Folie (2) integriert ist.

3. Allergietest-Vorrichtung (1) nach Anspruch 2, **dadurch gekennzeichnet, dass** in jedem beabstandeten Teil (4) eine vorzugsweise mit einem Deckel (9) verschließbare Befüllöffnung (8) zur Befüllung des Behälters (5) mit dem flüssigen Medium (M) angeordnet ist.

4. Allergietest-Vorrichtung (1) nach Anspruch 1, **dadurch gekennzeichnet, dass** der Behälter (5) durch einen mit dem beabstandeten Teil (4) der Folie (2) verbundenen Becher (10) gebildet ist.

5. Allergietest-Vorrichtung (1) nach einem der Ansprüche 1 bis 4, **dadurch gekennzeichnet, dass** zumindest jeder beabstandete Teil (4) der Folie (2), vorzugsweise die gesamte Folie (2) elastisch verformbar ausgebildet ist, und dass die Folie (2) vorzugsweise aus thermoplastischem Kunststoff oder Silikon, gebildet ist.

6. Allergietest-Vorrichtung (1) nach einem der Ansprüche 1 bis 5, **dadurch gekennzeichnet, dass** jeder beabstandete Teil (4) durch eine Ausstülpung (11) der Folie (2) gebildet ist, sodass bei der Anwendung ein vorzugsweise abgeschlossener Hohlraum (12) zur Haut (H) gebildet ist, wobei in der Ausstülpung (11) bevorzugt ein Entlüftungsloch (13) angeordnet ist.

7. Allergietest-Vorrichtung (1) nach einem der Ansprüche 1 bis 6, **dadurch gekennzeichnet, dass** an der, bei der Anwendung der Haut (H) zugewandten Seite des Behälters (5) und bzw. oder des Ventils (7) zumindest ein spitzes oder scharfes Element (14) zur lokalen und oberflächlichen Verletzung der Haut (H) vorgesehen ist, welches vorzugsweise einstückig mit dem Behälter (5) und bzw. oder dem Ventil (7) hergestellt ist.

8. Allergietest-Vorrichtung (1) nach einem der Ansprüche 1 bis 7, **dadurch gekennzeichnet, dass** jedes Ventil (7) durch einen im Wesentlichen zylindrischen Körper (15) mit zumindest einem Kanal (16) gebildet ist, welcher zylindrische Körper (15) in einer Schließposition des Ventils (7) die Öffnung (6) des Behälters (5) abdichtet und in einer von der Schließposition abweichenden Position den zumindest einen Kanal (16) zum Behälter (5) freigibt.

9. Allergietest-Vorrichtung (1) nach Anspruch 8, **dadurch gekennzeichnet, dass** der zumindest eine Kanal (16) durch zumindest einen im Inneren des zylindrischen Körpers (15) verlaufenden Kanal (16') mit einer Verbindung zu mindestens einer Öffnung (16") an der Mantelfläche des zylindrischen Körpers (15) gebildet ist, oder dass der zumindest eine Kanal (16) durch zumindest einen an der Oberfläche des zylindrischen Körpers (15) verlaufenden Kanal (16‴) gebildet ist.

10. Allergietest-Vorrichtung (1) nach einem der Ansprüche 8 bis 9, **dadurch gekennzeichnet, dass** am zylindrischen Körper (15) des Ventils (7) eine Dichtlippe (17) zur Abdichtung der Öffnung (6) des Behälters in der Schließposition integriert ist.

11. Allergietest-Vorrichtung (1) nach einem der Ansprüche 1 bis 10, **dadurch gekennzeichnet, dass** an der bei der Anwendung der Haut (H) zugewandten Seite des flachen Teils (3) der Folie (2) eine Klebefolie (18) mit einer Abziehfolie (19) angeordnet ist.

12. Allergietest-Vorrichtung (1) nach einem der Ansprüche 1 bis 11, **dadurch gekennzeichnet, dass** mehrere beabstandete Teile (4) einer Folie (2) in zumindest zwei Reihen (Rᵢ) angeordnet sind, wobei die Anzahl (nᵢ) der beabstandeten Teile (4) einer Folie (2) in jeder Reihe (Rᵢ) vorzugsweise unterschiedlich ist.

13. Allergietest-Vorrichtung (1) nach einem der Ansprüche 1 bis 12, **dadurch gekennzeichnet, dass** die Folie (2) zumindest teilweise aus transparentem Material, beispielsweise aus transparentem thermoplastischem Kunststoff oder transparentem Silikon, gebildet ist.

14. Allergietest-Vorrichtung (1) nach einem der Ansprüche 1 bis 13, **dadurch gekennzeichnet, dass** eine Transportsicherung (20) vorgesehen ist, mit im Wesentlichen zylinderförmigen Elementen (21), welche zur Anordnung an der Unterseite der Folie (2) zur Umfassung der Behälter (5) und Ventile (7) in den beabstandeten Teilen (4) ausgebildet ist, wobei an der Transportsicherung (20) bevorzugt ein Lineal (22), vorzugsweise mit halbkreisförmigen Aussparungen (23), zur Vermessung allergischer Reaktionen auf der Haut (H) angeordnet ist.

15. Allergietest-Vorrichtung (1) nach einem der Ansprüche 1 bis 14, **dadurch gekennzeichnet, dass** die Folie (2), der Behälter (5), das Ventil (7) und bzw. oder das zumindest eine spitze oder scharfe Elemente (14) zumindest teilweise aus biokompatiblem Material, beispielsweise aus Polyetheretherketon (PEEK), gebildet ist oder zumindest teilweise mit einer biokompatiblen Beschichtung, beispielsweise einer Parylene-Beschichtung, beschichtet ist.

## Claims

1. Allergy test device (1) for applying a liquid medium (M) onto or into the skin (H), with a film (2) with a flat part (3) for laying on the skin (H) and at least one part (4) spaced apart from the skin (H) during use with a container (5) for receiving the liquid medium (M), wherein each container (5) of each spaced part (4) has at least one closable opening (6), **characterised in that** each spaced part (4) is designed to be deformable in the direction of the flat part (3) of the film (2) and that a valve (7) is slidably arranged in the at least one opening (6) of each container (5), wherein the opening (6) of the container (5) is sealed in a closed position of the valve (7) and on deformation of the spaced part (4), the valve (7) is at least partly opened in a position deviating from the closed position.

2. Allergy test device (1) according to claim 1, **characterised in that** the container (5) is integral with the spaced part (4) of the film (2).

3. Allergy test device (1) according to Claim 2, **characterised in that** a filling opening (8), which can preferably be closed with a lid (9), for filling the container (5) with the liquid medium (M) is arranged in each spaced part (4).

4. Allergy test device (1) according to Claim 1, **characterised in that** the container (5) is formed by a cup (10) connected to the spaced part (4) of the film (2).

5. Allergy test device (1) according to any of Claims 1 to 4, **characterised in that** at least each spaced part (4) of the film (2), preferably the entire film (2), is designed to be elastically deformable, and that the film (2) is preferably made of thermoplastic synthetic material or silicone.

6. Allergy test device (1) according to any of Claims 1 to 5, **characterised in that** each spaced part (4) is formed by a protuberance (11) of the film (2), so that a preferably closed cavity (12) to the skin (H) is formed during use, wherein preferably a vent hole (13) is arranged in the protuberance (11).

7. Allergy test device (1) according to any of Claims 1 to 6, **characterised in that** on the side of the container (5) and or the valve (7) facing the skin (H) during use, at least one pointed or sharp element (14) is provided for local and superficial injury to the skin (H), which is preferably made in one piece with the container (5) and/or the valve (7).

8. Allergy test device (1) according to any of Claims 1 to 7, **characterised in that** each valve (7) is formed by a substantially cylindrical body (15) with at least one channel (16), which cylindrical body (15) in a closed position of the valve (7) seals the opening (6) of the container (5) and opens he at least one channel (16) to the container (5) in a position deviating from the closed position.

9. Allergy test device (1) according to Claim 8, **characterised in that** the at least one channel (16) is formed by at least one channel (16') running inside the cylindrical body (15) with a connection to at least one opening (16") on the lateral surface of the cylindrical body (15) or **in that** the at least one channel (16) is formed by at least one channel (16‴) running on the surface of the cylindrical body (15).

10. Allergy test device (1) according to any of Claims 8 to 9, **characterised in that** a sealing lip (17) for sealing the opening (6) of the container in the closed position is integrated on the cylindrical body (15) of the valve (7).

11. Allergy test device (1) according to any of Claims 1 to 10, **characterized in that** on the side of the flat part (3) of the film (2) facing the skin (H) during use there is an adhesive film (18) with a peel-off film (19).

12. Allergy test device (1) according to any of Claims 1 to 11, **characterised in that** several spaced parts (4) of a film (2) are arranged in at least two rows (Rᵢ), wherein the number (nᵢ) of the spaced parts (4) of a film (2) in each row (Rᵢ) is preferably different.

13. Allergy test device (1) according to any of Claims 1 to 12, **characterised in that** the film (2) is formed at least partly from transparent material, for example from transparent thermoplastic synthetic material or transparent silicone.

14. Allergy test device (1) according to any of Claims 1 to 13, **characterised in that** a transport lock (20) is provided, having essentially cylindrical elements (21), which is designed to be arranged on the underside of the film (2) for enclosing the containers (5) and valves (7) in the spaced parts (4), preferably a ruler (22), preferably with semi-circular recesses (23), is arranged on the transport lock (20) for measuring allergic reactions on the skin (H).

15. Allergy test device (1) according to any of Claims 1 to 14, **characterised in that** the film (2), the container (5), the valve (7) and/or the at least one pointed or sharp element (14) is at least partly formed from biocompatible material, for example polyether ether ketone (PEEK), or is at least partly coated with a biocompatible coating, such as a parylene coating.

## Revendications

1. Dispositif de test d'allergie (1) pour l'application d'un milieu liquide (M) sur ou dans la peau (H), comprenant une feuille (2) avec une partie plate (3) pour être appuyée sur la peau (H) et au moins une partie (4) espacée de la peau (H) lors de l'utilisation avec un récipient (5) pour recevoir le milieu liquide (M), chaque récipient (5) de chaque partie espacée (4) présentant au moins une ouverture (6) qui peut être fermée, **caractérisé, en ce que** chaque partie espacée (4) est réalisée de manière à pouvoir être déformée en direction de la partie plate (3) de la feuille (2) et **en ce qu'**une soupape (7) est disposée de manière à pouvoir être déplacée dans la au moins une ouverture (6) de chaque récipient (5), l'ouverture (6) du récipient (5) étant fermée de manière étanche dans une position de fermeture de la soupape (7) et la soupape (7) étant au moins partiellement ouverte dans une position différente de la position de fermeture lors de la déformation de la partie espacée (4).

2. Dispositif de test d'allergie (1) selon la revendication 1, **caractérisé en ce que** le récipient (5) est intégré dans la partie espacée (4) de la feuille (2).

3. Dispositif de test d'allergie (1) selon la revendication 2, **caractérisé en ce qu'**une ouverture de remplissage (8) qui peut être fermée, de préférence par un couvercle (9), se trouve dans chaque partie (4) espacée pour remplir le récipient (5) avec le milieu liquide (M).

4. Dispositif de test d'allergie (1) selon la revendication 1, **caractérisé en ce que** le récipient (5) est formé par une coupe (10) reliée à la partie espacée (4) de la feuille (2).

5. Dispositif de test d'allergie (1) selon l'une des revendications 1 à 4, **caractérisé en ce qu'**au moins chaque partie espacée (4) de la feuille (2), de préférence la feuille entière (2), est réalisée de manière à pouvoir être déformée élastiquement, et **en ce que** la feuille (2) est formée de préférence d'une matière thermoplastique ou de silicone.

6. Dispositif de test d'allergie (1) selon l'une des revendications 1 à 5, **caractérisé en ce que** chaque partie espacée (4) est formée par une protubérance (11) de la feuile (2), de sorte que, lors de l'utilisation, un espace creux (12), de préférence fermé, est formé par rapport à la peau (H), un trou d'aération (13) étant de préférence disposé dans ladite protubérance (11).

7. Dispositif de test d'allergie (1) selon l'une des revendications 1 à 6, **caractérisé en ce qu'**au moins un élément pointu ou tranchant (14) est disposé sur le côté du récipient (5) et/ou de la valve (7) tourné vers la peau (H) lors de l'utilisation pour blesser localement et superficiellement la peau (H), ledit élément étant de préférence fait en une seule pièce avec le récipient (5) et/ou la valve (7).

8. Dispositif de test d'allergie (1) selon l'une des revendications 1 à 7, **caractérisé en ce que** chaque valve (7) est formée par un corps sensiblement cylindrique (15) avec au moins un canal (16), lequel corps cylindrique (15), dans une position de fermeture de la valve (7), ferme hermétiquement l'ouverture (6) du récipient (5) et, dans une position différente de la position de fermeture, débloque ledit au moins un canal (16) ménant dans le récipient (5).

9. Dispositif de test d'allergie (1) selon la revendication 8, **caractérisé en ce que** ledit au moins un canal (16) est formé par au moins un canal (16') s'étendant à l'intérieur du corps cylindrique (15) avec une connexion à au moins une ouverture (16") sur la surface d'enveloppe du corps cylindrique (15), ou **en ce que** ledit au moins un canal (16) est formé par au moins un canal (16'") s'étendant à la surface du corps cylindrique (15).

10. Dispositif de test d'allergie (1) selon l'une des revendications 8 à 9, **caractérisé en ce qu'**une lèvre d'étanchéité (17) est intégrée dans le corps cylindrique (15) de la valve (7) pour assurer l'étanchéité de l'ouverture (6) du récipient en position de fermeture.

11. Dispositif de test d'allergie (1) selon l'une des revendications 1 à 10, **caractérisé en ce que** une feuille adhésive (18) avec une feuille détachable (19) est disposée sur le côté de la partie plate (3) de la feuille (2) qui est tourné vers la peau (H) lors de l'utilisation.

12. Dispositif de test d'allergie (1) selon l'une des revendications 1 à 11, **caractérisé en ce que** plusieurs parties espacées (4) d'une feuille (2) sont disposées en au moins deux rangées (Rᵢ), le nombre (nᵢ) de parties espacées (4) d'une feuille (2) étant de préférence différent dans chaque rangée (Rᵢ).

13. Dispositif de test d'allergie (1) selon l'une des revendications 1 à 12, **caractérisé en ce que** la feuille (2) est formée au moins partiellement d'un matériau transparent, par exemple d'une matière thermoplastique transparente ou d'une silicone transparente.

14. Dispositif de test d'allergie (1) selon l'une des revendications 1 à 13, **caractérisé en ce qu'**un dispositif de sécurité de transport (20) est prévu, avec des éléments (21) essentiellement cylindriques, qui est conçu pour être disposé sur la face inférieure de la feuille (2) pour entourer les récipients (5) et les valves (7) dans les parties (4) espacées, une règle (22), de préférence avec des évidements (23) semi-circulaires, étant disposée sur le dispositif de sécurité de transport (20) pour mesurer les réactions allergiques sur la peau (H).

15. Dispositif de test d'allergie (1) selon l'une des revendications 1 à 14, **caractérisé en ce que** la feuille (2), le récipient (5), la valve (7) et/ou ledit au moins un élément pointu ou tranchant (14) est formé au moins partiellement d'un matériau biocompatible, par exemple de polyétheréthercétone (PEEK), ou est au moins partiellement revêtu d'un revêtement biocompatible, par exemple un revêtement de parylène.
